# EUROPEAN PATENT APPLICATION

(11) **EP 1 923 393 A1**
(43) Date of publication of application: **21.05.2008**
(21) Application number: 06124269.9
(22) Date of filing: 17.11.2006
(51) Int. Cl.: C07D 451/10, A61K 31/46, A61P 11/00

(54) **Crystalline form of tiotropium bromide and urea**

(71) Applicant: Boehringer Ingelheim Pharma GmbH & Co. KG, 55216 Ingelheim (DE)
(72) Inventor: The designation of the inventor has not yet been filed
(74) Representative: Hammann, Heinz

(57) **Abstract**

The invention relates to a new crystalline form of tiotropium bromide, processes for preparing it and its use for preparing a pharmaceutical composition for the treatment of respiratory complaints, particularly for the treatment of COPD (chronic obstructive pulmonary disease) and asthma.

## Description

The invention relates to a new crystalline form of tiotropium bromide, processes for preparing it and its use for preparing a pharmaceutical composition for the treatment of respiratory complaints, particularly for the treatment of COPD (chronic obstructive pulmonary disease) and asthma.

### Background to the invention

Tiotropium bromide is known from European Patent Application EP 418 716 A1 and has the following chemical structure:

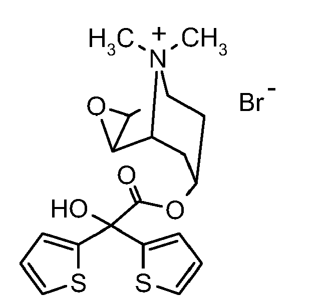

Tiotropium bromide is a highly effective anticholinergic with a long-lasting effect, which may be used to treat respiratory complaints, particularly COPD (chronic obstructive pulmonary disease) and asthma. By tiotropium is meant the free ammonium cation.

Tiotropium bromide is preferably administered by inhalation. Suitable inhalable powders packed into appropriate capsules (inhalettes) may be used. Alternatively, it may be administered by the use of suitable inhalable aerosols. These also include powdered inhalable aerosols which contain, for example, HFA134a, HFA227 or mixtures thereof as propellent gas.

The correct manufacture of the abovementioned compositions which are suitable for use for the administration of a pharmaceutically active substance by inhalation is based on various parameters which are connected with the nature of the active substance itself. In pharmaceutical compositions which are used like tiotropium bromide in the form of inhalable powders or inhalable aerosols, the crystalline active substance is used in ground (micronised) form for preparing the formulation. Since the pharmaceutical quality of a pharmaceutical formulation requires that the active substance should always have the same crystalline modification, the stability and properties of the crystalline active substance are subject to stringent requirements from this point of view as well.

The aim of the invention is therefore to provide a new crystal form of the compound tiotropium bromide which meets the high demands mentioned above that are made of any pharmaceutically active substance.

### Detailed description of the invention

It has surprisingly been found that, starting from the monohydrate of tiotropium bromide, which was described in the prior art for the first time in WO 02/30928, a novel crystal modification of tiotropium bromide may be obtained in form of a co-crystal with urea. Surprisingly this co-crystal shows only very little hygroscopical behaviour, thereby representing a crystalline form of tiotropium bromide that is relatively stable towards the influence of moisture and humidity.

Accordingly, in the present invention relates to this novel tiotropium bromide - urea co-crystal. In the novel co-crystal the components tiotropium bromide and urea are present in an almost stoichiometric ratio. Therefore, the present invention relates to a tiotropium bromide - urea co-crystal in which the components tiotropium bromide and urea are present in a ratio of about 1:1.

The novel co-crystal is characterized by a sharp endothermic event at about 174 °C as determined via DSC.

The novel co-crystal is characterized by a X-ray powder diagram with characteristic values at d= 12.48 Å; 6.43 Å; 5.04 Å; 4.08 Å; 3.99 Å; 3.71 Å; 3.53 Å and 3.38 Å; *inter alia.* It is in particular characterized by the most intensive values d= 5.041 Å; 3.99 Å and 3.53 Å; *inter alia.*

The present invention also relates to the use of the novel co-crystal according to the invention for preparing a pharmaceutical composition for the treatment of respiratory complaints, particularly for the treatment of COPD and/or asthma.

The present invention also relates to methods for the preparation of the crystalline tiotropium bromide forms according to the inventions.

In another aspect the present invention relates to a method of preparing the new co-crystal of tiotropium bromide characterized in that crystalline tiotropium bromide monohydrate (as known from WO 02/30928) is milled in a 1:1 molar ratio with urea to lead to an amorphous mixture which is then slurried in a suitable solvent, preferably at elevated temperature. The residual solid material is recovered by filtration and dried under ambient conditions. Suitable solvent are preferably selected from among ethyl acetate, tetraline or hexane, ethylacetate being particular preferred. Preferably the formation of the co-crystal occurs at an elevated temperature of about 50 °C.

The Examples that follow serve to illustrate the present invention still further, without restricting the scope of the invention to the embodiments by way of example that follow.

### Example of synthesis

100 mg of Tiotropiumbromide-Monohydrate are milled in a ball mill (Retsch MM200) for 60 minutes at 20 Hz in a 1:1 molar ratio with urea (12.7 mg, CH₄N₂O, M = 60.06) until a completely amorphous mixture of both components is obtained. This mixture is slurried in ethyl acetate at room temperature for about 4 hours and than heated up to 50 °C for an additional 4 hours. This procedure is repeated two additional times resulting in a total treatment of the mixture for 24 hours. Afterwards the residual solid material is recovered by filtration and dried over night under ambient conditions.

### Analytics

The obtained crystals were analysed by X-Ray powder diffraction (= XRPD), thermal analysis (DSC), and ¹H-NMR indicating that a new crystalline form, namely a co-crystal of Tiotropiumbromide with urea has been formed.

### X-ray powder diffraction

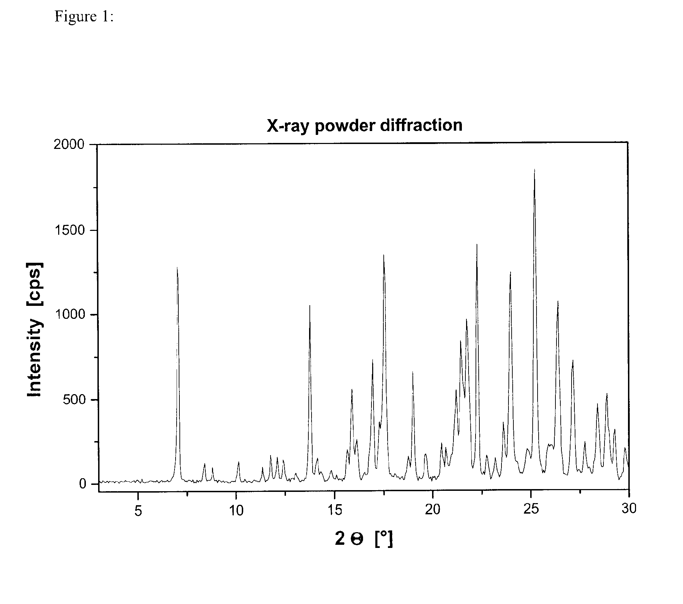

X-ray powder diffraction patterns for the samples were acquired on a Siemens D5000 diffractometer using CuKα radiation (40kV, 40mA), θ-θ goniometer, automatic divergence and receiving slits, a graphite secondary monochromator and a scintillation counter. The data were collected over an angular range of 2° to 42° 2θ in continuous scan mode using a step size of 0.02° 2θ and a step time of 1 second.
Samples run under ambient conditions were prepared as flat plate specimens using powder as received without grinding. Approximately, 25-50 mg of the sample was gently packed into 12 mm diameter, 0.5 mm deep cavities cut into polished, zero-background (510) silicon wafers (The Gem Dugout, 1652 Princeton Drive, Pennsylvania State College, PA 16803, USA). All specimens were analysed both stationary and rotated in their own plane during analysis. A further specimen was tested using silicon powder as an internal standard to correct for any peak displacement.
A representative diagram in shown in figure 1 and a list of the characteristic diffraction peaks including normalised intensities is shown in table 1 below.

**Table 1: X-ray powder reflections (up to 30 °2Θ) and intensities (normalized) of Tiotropiumbromide/urea co-crystal**

| **2Θ [°]** | **d [A]** | **I/Iₒ [%]** |
|---|---|---|
| 7.08 | 12.48 | 69 |
| 8.41 | 10.50 | 5 |
| 8.79 | 10.05 | 4 |
| 10.15 | 8.70 | 6 |
| 11.37 | 7.78 | 4 |
| 11.79 | 7.50 | 8 |
| 12.09 | 7.31 | 7 |
| 12.45 | 7.11 | 7 |
| 13.77 | 6.43 | 50 |
| 14.15 | 6.25 | 8 |
| 14.81 | 5.98 | 4 |
| 15.66 | 5.65 | 11 |
| 15.88 | 5.58 | 28 |
| 16.12 | 5.49 | 13 |
| 16.93 | 5.23 | 37 |
| 17,30 | 5,12 | 14 |
| 17.58 | 5.04 | 72 |
| 18.75 | 4.73 | 8 |
| 19.01 | 4.66 | 33 |
| 19.65 | 4.51 | 10 |
| 20.47 | 4.33 | 12 |
| 20.69 | 4.29 | 12 |
| 21.18 | 4.19 | 29 |
| 21.45 | 4.14 | 45 |
| 21.75 | 4.08 | 53 |
| 22.28 | 3.99 | 73 |
| 22.75 | 3.91 | 9 |
| 23.19 | 3.83 | 7 |
| 23.63 | 3.76 | 18 |
| 23.97 | 3.71 | 68 |
| 24.83 | 3.58 | 11 |
| 25.22 | 3.53 | 100 |
| 25.96 | 3.43 | 11 |
| 26,04 | 3,42 | 13 |
| 26.37 | 3.38 | 57 |
| 27.13 | 3.28 | 40 |
| 27.73 | 3.21 | 12 |
| 28.42 | 3.14 | 25 |
| 28.87 | 3.09 | 27 |
| 29.27 | 3.05 | 17 |
| 29.80 | 3.00 | 11 |

### Thermal analysis - Differential Scanning Calorimetry (DSC)

DSC data was collected on a TA instrument Q1000 equipped with a 50 position autosampler. The enthalphy of fusion and temperature calibration standard was indium.

Samples were heated at a rate of 10 °C/min between 10 and 230 °C. A nitrogen purge at 30 cm³/min was maintained over the sample.
Between 1 and 3 mg of sample was used, unless otherwise stated, and all samples were crimped in hermetically sealed aluminium pans.

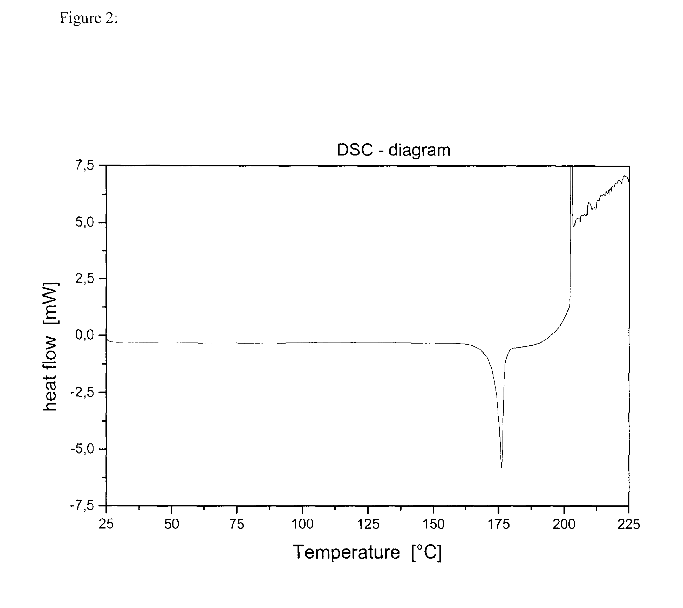

The DSC - trace of the urea co-crystal with Tiotropiumbromide shows a sharp endothermic event at ca. 174 °C indicating melting of this material. Above 200 °C thermal decomposition is observed. The obtained DSC-diagram is depicted in figure 2.

### NMR - analysis

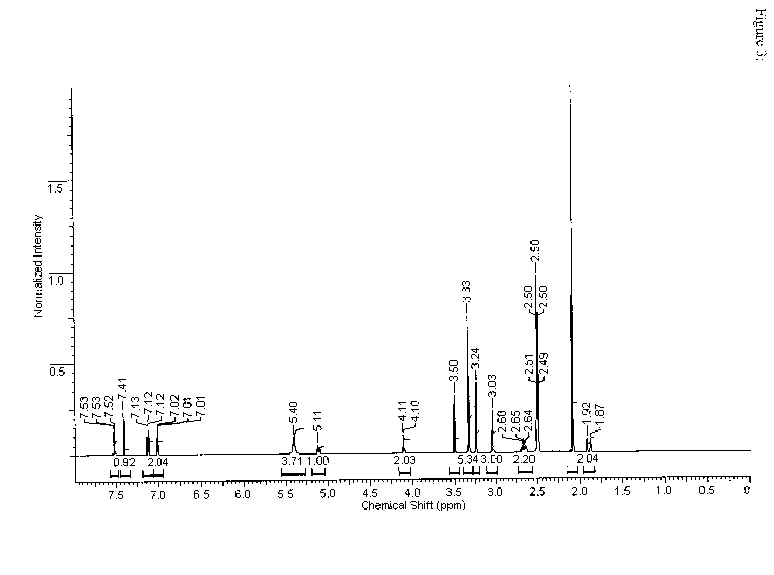

In order to get an idea on the stoichiometry of the obtained co-crystal ¹H-NMR spectra were recorded on a Bruker 400 MHz spectrometer. The samples were dissolved in *d₆-*DMSO for analysis. The corresponding spectrum is shown in figure 3. In addition to the characteristic ¹H-NMR signals of tiotropium there is a signal at 5.40 ppm which is indicative of urea. Integration of this signal shows that the co-crystal has a stoichiometry which is close to 1:1 (0.93 eq of urea).

### Formulations containing the tiotropium bromide form according to the invention

The crystalline tiotropium bromide form according to the invention is particularly well suited to the preparation of, for example, pharmaceutical formulations for administration by inhalation such as inhalable powders or for example propellant-containing aerosol formulations, particularly inhalable powders and propellant-containing aerosol suspensions. These pharmaceutical formulations or compositions may contain in addition to the crystalline tiotropium form according to the invention one or more additional active ingredients selected from among betamimetics, EGFR inhibitors, PDEIV-inhibitors, steroids, and LTD4 antagonists, optionally together with a pharmaceutically acceptable excipient.

### Inhalable powders

The present invention also relates to inhalable powder containing 0.001 to 3 % tiotropium in the form of the crystalline tiotropium bromide forms according to the invention combined with a physiologically acceptable excipient. By tiotropium is meant the ammonium cation.
Inhalable powders which contain 0.01 to 2 % tiotropium are preferred according to the invention. Particularly preferred inhalable powders contain tiotropium in an amount from about 0.03 to 1 %, preferably 0.05 to 0.6 %, particularly preferably 0.06 to 0.3 %. Of particular importance according to the invention, finally, are inhalable powders which contain about 0.08 to 0.22 % tiotropium.
The amounts of tiotropium specified above are based on the amount of tiotropium cation contained.

The excipients that are used for the purposes of the present invention are prepared by suitable grinding and/or screening using current methods known in the art. The excipients used according to the invention may also be mixtures of excipients which are obtained by mixing excipient fractions of different mean particle sizes.

Examples of physiologically acceptable excipients which may be used to prepare the inhalable powders for use in the inhalettes according to the invention include monosaccharides (e.g. glucose, fructose or arabinose), disaccharides (e.g. lactose, saccharose, maltose, trehalose), oligo- and polysaccharides (e.g. dextrans, dextrins, maltodextrin, starch, cellulose), polyalcohols (e.g. sorbitol, mannitol, xylitol), cyclodextrins (e.g. α-cyclodextrin, β-cyclodextrin, χ-cyclodextrin, methyl-β-cyclodextrin, hydroxypropyl-β-cyclodextrin), amino acids (e.g. arginine hydrochloride) or salts (e.g. sodium chloride, calcium carbonate), or mixtures thereof. Preferably, mono- or disaccharides are used, while the use of lactose or glucose is preferred, particularly, but not exclusively, in the form of their hydrates. For the purposes of the invention, lactose is the particularly preferred excipient.

Within the scope of the inhalable powders according to the invention the excipients have a maximum average particle size of up to 250 µm, preferably between 10 and 150 µm, most preferably between 15 and 80 µm. It may sometimes seem appropriate to add finer excipient fractions with an average particle size of 1 to 9 µm to the excipients mentioned above. These finer excipients are also selected from the group of possible excipients listed hereinbefore. The average particle size may be determined using methods known in the art (cf. for example WO 02/30389, paragraphs A and C). Finally, in order to prepare the inhalable powders according to the invention, micronised crystalline tiotropium bromide anhydrate, which is preferably characterised by an average particle size of 0.5 to 10µm, particularly preferably from 1 to 5µm, is added to the excipient mixture (cf. for example WO 02/30389, paragraph B). Processes for grinding and micronising active substances are known from the prior art.

If no specifically prepared excipient mixture is used as the excipient, it is particularly preferable to use excipients which have a mean particle size of 10 - 50 µm and a 10 % fine content of 0.5 to 6 µm.

By average particle size is meant here the 50 % value of the volume distribution measured with a laser diffractometer using the dry dispersion method . The average particle size may be determined using methods known in the art (cf. for example WO 02/30389, paragraphs A and C). Analogously, the 10% fine content in this instance refers to the 10% value of the volume distribution measured using a laser diffractometer. In other words, for the purposes of the present invention, the 10% fine content denotes the particle size below which 10% of the quantity of particles is found (based on the volume distribution).

The percentages given within the scope of the present invention are always percent by weight, unless specifically stated to the contrary.

In particularly preferred inhalable powders the excipient is characterised by a mean particle size of 12 to 35 µm, particularly preferably from 13 to 30 µm.
Also particularly preferred are those inhalable powders wherein the 10 % fine content is about 1 to 4 µm, preferably about 1.5 to 3 µm.

The inhalable powders according to the invention are characterised, in accordance with the problem on which the invention is based, by a high degree of homogeneity in the sense of the accuracy of single doses. This is in the region of < 8 % , preferably < 6 % , most preferably < 4 %.

After the starting materials have been weighed out the inhalable powders are prepared from the excipient and the active substance using methods known in the art. Reference may be made to the disclosure of WO 02/30390, for example. The inhalable powders according to the invention may accordingly be obtained by the method described below, for example. In the preparation methods described hereinafter the components are used in the proportions by weight described in the above-mentioned compositions of the inhalable powders.

First, the excipient and the active substance are placed in a suitable mixing container. The active substance used has an average particle size of 0.5 to 10 µm, preferably 1 to 6 µm, most preferably 2 to 5 µm. The excipient and the active substance are preferably added using a sieve or a granulating sieve with a mesh size of 0.1 to 2 mm, preferably 0.3 to 1 mm, most preferably 0.3 to 0.6 mm. Preferably, the excipient is put in first and then the active substance is added to the mixing container. During this mixing process the two components are preferably added in batches. It is particularly preferred to sieve in the two components in alternate layers. The mixing of the excipient with the active substance may take place while the two components are still being added. Preferably, however, mixing is only done once the two components have been sieved in layer by layer.

The present invention also relates to the use of the inhalable powders according to the invention for preparing a pharmaceutical composition for the treatment of respiratory complaints, particularly for the treatment of COPD and/or asthma.

The inhalable powders according to the invention may for example be administered using inhalers which meter a single dose from a reservoir by means of a measuring chamber (e.g. according to US 4570630A) or by other means (e.g. according to DE 36 25 685 A). Preferably, however, the inhalable powders according to the invention are packed into capsules (to make so-called inhalettes), which are used in inhalers such as those described in WO 94/28958, for example.

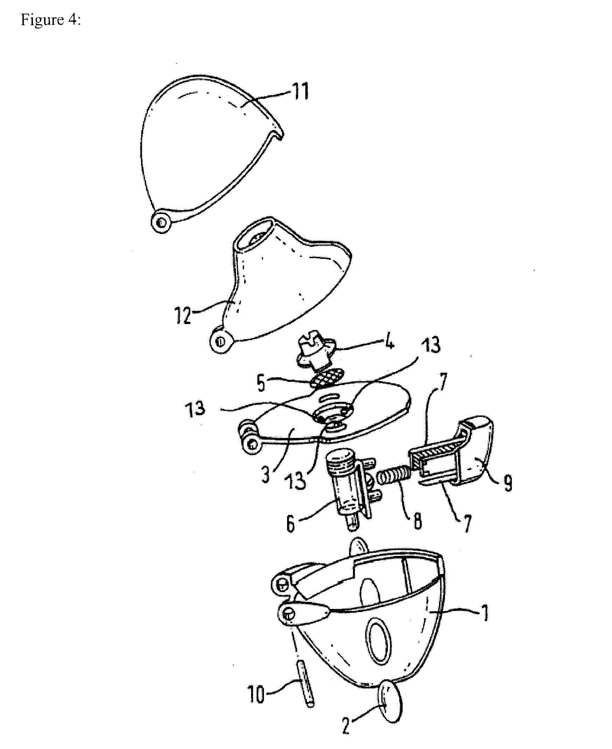

Most preferably, the capsules containing the inhalable powder according to the invention are administered using an inhaler as shown in Figure 4. This inhaler is characterised by a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, and a mouthpiece 12 which is connected to the housing 1, the deck 3 and a cover 11 via a spindle 10 to enable it to be flipped open or shut and airholes 13 for adjusting the flow resistance.

The present invention further relates to the use of the inhalable powders containing one or several, preferably one of the crystalline tiotropium bromide forms according to the invention for preparing a pharmaceutical composition for treating respiratory complaints, particularly for the treatment of COPD and/or asthma, characterised in that the inhaler described above and shown in Figure 15 is used.

For administering the inhalable powders containing the crystalline tiotropium bromide forms according to the invention using powder-filled capsules it is particularly preferred to use capsules the material of which is selected from among the synthetic plastics, most preferably selected from among polyethylene, polycarbonate, polyester, polypropylene and polyethylene terephthalate. Particularly preferred synthetic plastic materials are polyethylene, polycarbonate or polyethylene terephthalate. If polyethylene is used as one of the capsule materials which is particularly preferred according to the invention, it is preferable to use polyethylene with a density of between 900 and 1000 kg/m³, preferably 940 - 980 kg/m³, more preferably about 960 - 970 kg/m³ (high density polyethylene).
The synthetic plastics according to the invention may be processed in various ways using manufacturing methods known in the art. Injection moulding of the plastics is preferred according to the invention. Injection moulding without the use of mould release agents is particularly preferred. This method of production is well defined and is characterised by being particularly reproducible.

In another aspect the present invention relates to the abovementioned capsules which contain the abovementioned inhalable powder according to the invention. These capsules may contain about 1 to 20 mg, preferably about 3 to 15 mg, most preferably about 4 to 12 mg of inhalable powder. Preferred formulations according to the invention contain 4 to 6 mg of inhalable powder. Of equivalent importance according to the invention are capsules for inhalation which contain the formulations according to the invention in an amount of from 8 to 12 mg.

The present invention also relates to an inhalation kit consisting of one or more of the above capsules characterised by a content of inhalable powder according to the invention in conjunction with the inhaler according to Figure 15.

The present invention also relates to the use of the abovementioned capsules characterised by a content of inhalable powder according to the invention, for preparing a pharmaceutical composition for treating respiratory complaints, especially for treating COPD and/or asthma.

Filled capsules which contain the inhalable powders according to the invention are produced by methods known in the art, by filling the empty capsules with the inhalable powders according to the invention.

### Examples of inhalable powders according to the invention

The following Examples serve to illustrate the present invention in more detail without restricting the scope of the invention to the exemplifying embodiments that follow.

### Active substance

The crystalline tiotropium bromide forms according to the invention are used to produce the inhalable powders according to the invention. The micronisation of these forms may be carried out analogously to methods known in the art (cf for example WO 03/078429 A1). Where reference is made within the scope of the present invention to the mean particle size of the crystalline tiotropium bromide forms according to the invention, this is determined using methods of measurement known in the art (cf for example WO 03/078429 A1, para. D.2).

### Excipient:

In the Examples that follow lactose-monohydrate is used as excipient. It may be obtained for example from Borculo Domo Ingredients, Borculo/NL under the product name *Lactochem Extra Fine Powder.* The specifications according to the invention for the particle size and specific surface area are met by this grade of lactose. For example, in the Examples that follow, batches of lactose were used having the following specifications:

### Preparation of the powder formulations :

### Apparatus

The following machines and equipment, for example, may be used to prepare the inhalable powders:
Mixing container or powder mixer: Turbulamischer 2 L, Type 2C; made by Willy A. Bachofen AG, CH-4500 Basel
Hand-held screen: 0.135 mm mesh size

The empty inhalation capsules may be filled with inhalable powders containing tiotropium by hand or mechanically. The following equipment may be used.

### Capsule filling machine:

MG2, Type G100, manufacturer: MG2 S.r.1, I-40065 Pian di Macina di Pianoro (BO), Italy

### Formulation Examples:

Formulation Example 1 - Powder mixture :
To prepare the powder mixture, 299.39 g of excipient and 0.61 g of the micronised co-crystal according to the invention are used.

About 40-45 g of excipient are placed in a suitable mixing container through a hand-held screen with a mesh size of 0.315 mm. Then the co-crystal according to the invention in batches of about 90-110 mg and excipient in batches of about 40-45 g are screened in in alternate layers. The excipient and active substance are added in 7 and 6 layers, respectively.

Having been screened in, the ingredients are then mixed (mixing speed 900 rpm). The final mixture is passed twice more through a hand-held screen and then mixed again at 900 rpm.

Using the method described in formulation Example 1 it is possible to obtain inhalable powders which when packed into suitable plastic capsules may be used to produce the following capsules for inhalation, for example:

### Formulation Example 2:

| | |
|---|---|
| tiotropium bromide - urea co-crystal: | 0.0113 mg |
| lactose monohydrate: | 5.4887 mg |
| capsule: | 100.0 mg |
| Total: | 105.5 mg |

### Formulation Example 3:

| | |
|---|---|
| tiotropium bromide - urea co-crystal: | 0.0225 mg |
| lactose monohydrate: | 5.4775 mg |
| polyethylene capsules: | 100.0 mg |
| Total: | 105.5 mg |

### Formulation Example 4:

| | |
|---|---|
| tiotropium bromide - urea co-crystal: | 0.0056 mg |
| lactose monohydrate: | 5.4944 mg |
| polyethylene capsules: | 100.0 mg |
| Total: | 105.5 mg |

### Propellant-containing aerosol suspensions

The co-crystal according to the invention may optionally also be administered in the form of propellant-containing inhalable aerosols. Aerosol suspensions are particularly suitable for this.

The present invention therefore also relates to suspensions of the co-crystal according to the invention in the propellent gases HFA 227 and/or HFA 134a, optionally combined with one or more other propellent gases, preferably selected from the group consisting of propane, butane, pentane, dimethylether, CHClF₂, CH₂F₂, CF₃CH₃, isobutane, isopentane and neopentane.

According to the invention those suspensions which contain as propellent gas only HFA 227, a mixture of HFA 227 and HFA 134a or only HFA 134a are preferred.
If a mixture of the propellent gases HFA 227 and HFA 134a is used in the suspension formulations according to the invention, the weight ratios in which these two propellent gas components are used are freely variable.
If one or more other propellent gases, selected from the group consisting of propane, butane, pentane, dimethylether, CHClF₂, CH₂F₂, CF₃CH₃, isobutane, isopentane and neopentane are used in addition to the propellent gases HFA 227 and/or HFA 134a in the suspension formulations according to the invention, the amount of this additional propellent gas component is preferably less than 50 %, preferably less than 40%, particularly preferably less than 30%.

The suspensions according to the invention preferably contain an amount of tiotropium bromide form such that the amount of tiotropium cation is between 0.001 and 0.8%, preferably between 0.08 and 0.5%, and particularly preferably between 0.2 and 0.4% according to the invention. Unless stated to the contrary, the percentages given within the scope of the present invention are always percent by weight.

In some cases, the term suspension formulation is used within the scope of the present invention instead of the term suspension. The two terms are to be regarded as equivalent within the scope of the present invention.

The propellant-containing inhalable aerosols or suspension formulations according to the invention may also contain other constituents such as surface-active agents (surfactants), adjuvants, antioxidants or flavourings.

The surface-active agents (surfactants) optionally present in the suspensions according to the invention are preferably selected from the group consisting of Polysorbate 20, Polysorbate 80, Myvacet 9-45, Myvacet 9-08, isopropyl myristate, oleic acid, propyleneglycol, polyethyleneglycol, Brij, ethyl oleate, glyceryl trioleate, glyceryl monolaurate, glyceryl monooleate, glyceryl monostearate, glyceryl monoricinoleate, cetylalcohol, sterylalcohol, cetylpyridinium chloride, block polymers, natural oil, ethanol and isopropanol. Of the above-mentioned suspension adjuvants Polysorbate 20, Polysorbate 80, Myvacet 9-45, Myvacet 9-08 or isopropyl myristate are preferably used. Myvacet 9-45 or isopropyl myristate are most preferably used.

If the suspensions according to the invention contain surfactants these are preferably used in an amount of 0.0005 - 1 %, particularly preferably 0.005 - 0.5 %.

The adjuvants optionally contained in the suspensions according to the invention are preferably selected from the group consisting of alanine, albumin, ascorbic acid, aspartame, betaine, cysteine, phosphoric acid, nitric acid, hydrochloric acid, sulphuric acid and citric acid. Ascorbic acid, phosphoric acid, hydrochloric acid or citric acid are preferably used, while hydrochloric acid or citric acid is most preferably used.

If adjuvants are present in the suspensions according to the invention, these are preferably used in an amount of 0.0001-1.0 %, preferably 0.0005-0.1 %, particularly preferably 0.001-0.01 %, while an amount of 0.001-0.005 % is particularly important according to the invention.

The antioxidants optionally contained in the suspensions according to the invention are preferably selected from the group consisting of ascorbic acid, citric acid, sodium edetate, editic acid, tocopherols, butylhydroxytoluene, butylhydroxyanisol and ascorbylpalmitate, while tocopherols, butylhydroxytoluene, butylhydroxyanisol or ascorbylpalmitate are preferably used.

The flavourings optionally contained in the suspensions according to the invention are preferably selected from the group consisting of peppermint, saccharine, Dentomint, aspartame and ethereal oils (for example cinnamon, aniseed, menthol, camphor), of which peppermint or Dentomint® are particularly preferred.

With a view to administration by inhalation it is essential to provide the active substance in finely divided form. For this purpose, the co-crystal according to the invention is obtained in finely divided form using methods known in the prior art. Methods of micronising active substances are known in the art. Preferably after micronising the active substance has a mean particle size of 0.5 to 10 µm, preferably 1 to 6 µm, particularly preferably 1.5 to 5 µm. Preferably at least 50%, preferably at least 60%, particularly preferably at least 70% of the particles of active substance have a particle size which is within the size ranges mentioned above. Particularly preferably at least 80%, most preferably at least 90% of the particles of active substance have a particle size which is within the size ranges mentioned above.

The suspensions according to the invention may be prepared using methods known in the art. For this, the constituents of the formulation are mixed with the propellent gas or gases (optionally at low temperatures) and filled into suitable containers.

The above-mentioned propellant-containing suspensions according to the invention may be administered using inhalers known in the art (pMDIs = pressurized metered dose inhalers). Accordingly, in another aspect, the present invention relates to pharmaceutical compositions in the form of suspensions as hereinbefore described combined with one or more inhalers suitable for administering these suspensions. Moreover the present invention relates to inhalers, characterised in that they contain the propellant-containing suspensions according to the invention described hereinbefore.

The present invention also relates to containers (cartridges) which when fitted with a suitable valve can be used in a suitable inhaler and which contain one of the above-mentioned propellant-containing suspensions according to the invention. Suitable containers (cartridges) and processes for filling these cartridges with the propellant-containing suspensions according to the invention are known in the art.

In view of the pharmaceutical activity of tiotropium the present invention also relates to the use of the suspensions according to the invention for preparing a pharmaceutical composition for inhalation or nasal administration, preferably for preparing a pharmaceutical composition for inhalative or nasal treatment of diseases in which anticholinergics may develop a therapeutic benefit.

Particularly preferably the present invention also relates to the use of the suspensions according to the invention for preparing a pharmaceutical composition for the inhalative treatment of respiratory complaints, preferably asthma or COPD.

The Examples that follow serve to illustrate the present invention in more detail, by way of example, without restricting it to their contents.

### Examples of aerosol suspension formulations

Suspensions containing other ingredients in addition to active substance and propellent gas:

### Formulation Example 5:

| **constituents** | **concentration [% w/w]** |
|---|---|
| tiotropium bromide - urea co-crystal | 0.04 |
| oleic acid | 0.005 |
| HFA-227 | 99.955 |

### Formulation Example 6:

| **constituents** | **concentration [% w/w]** |
|---|---|
| tiotropium bromide - urea co-crystal | 0.02 |
| oleic acid | 0.01 |
| HFA-227 | 60.00 |
| HFA-134a | 39.97 |

### Formulation Example 7:

| **constituents** | **concentration [% w/w]** |
|---|---|
| tiotropium bromide - urea co-crystal | 0.02 |
| isopropylmyristate | 1.00 |
| HFA-227 | 98.98 |

### Formulation Example 8:

| **constituents** | **concentration [% w/w]** |
|---|---|
| tiotropium bromide - urea co-crystal | 0.02 |
| isopropylmyristate | 1.00 |
| HFA-134a | 98.98 |

## Claims

1. Crystalline form consisting of tiotropium bromide and urea.

2. Crystalline form according to claim 1, wherein tiotropium bromide and urea are present in an amount of about 1:1.

3. Crystalline form according to claim 1, which is **characterized by** an endothermic event at about 174 °C as determined via DSC.

4. Crystalline form according to claim 1, which is **characterized by** a X-ray powder diagram with characteristic values at d= 5.04 Å; 3.991 Å and 3.53 Å ; *inter alia.*

5. Pharmaceutical composition, **characterised in that** it contains a tiotropium form according to one of claims 1-4.

6. Pharmaceutical composition according to claim 5, **characterised in that** it contains a tiotropium form according to one of claims 1-4 in combination with one or more active ingredients selected from among betamimetics, EGFR inhibitors, PDEIV-inhibitors, steroids, and LTD4 antagonists, optionally together with a pharmaceutically acceptable excipient.

7. Use of a tiotropium form according to one of claims 1-4 for preparing a pharmaceutical composition for the treatment of respiratory complaints, preferably asthma or COPD.

8. Method of preparing the crystalline anhydrous tiotropium bromide according to claim 1, **characterized in** crystalline tiotropium bromide monohydrate is milled in a 1:1 molar ratio with urea to lead to an amorphous mixture which is then slurried in a suitable solvent.

9. Use of crystalline tiotropium bromide monohydrate as the starting material for the manufacture of the crystalline tiotropium form according to claim 1.
